# FASCICULE DE BREVET EUROPEEN

(11) **EP 0 639 586 B1**
(45) Date de publication et mention de la délivrance du brevet: **21.03.2001**
(21) Numéro de dépôt: 94401268.1
(22) Date de dépôt: 08.06.1994
(51) Int. Cl.: C07K 5/06, C07K 9/00, C07F 9/09, A61K 38/55, A61K 31/66

(54) **Dérivés d'acide phosphonique, leur procédé de préparation et les compositions pharmaceutiques qui les contiennent**
Derivate der Phosphonsäure, Verfahren zu ihrer Herstellung und pharmazeutische Zusammensetzungen die sie enthalten
Phosphonic acid derivatives, process for their preparation and pharmaceutical compositions comprising them

(30) Priorité: 30.06.1993 FR 9307927
(43) Date de publication de la demande: 22.02.1995
(73) Titulaire: ADIR ET COMPAGNIE, 92415 Courbevoie Cédex (FR)
(72) Inventeur: de nanteuil, Guillaume, F-92150 Suresnes (FR); Remond, Georges, F-78000 Versailles (FR); Verbeuren, Tony, F-78540 Vernouillet (FR)

(56) Documents cités:
- WO-A-93/11154
- DE-A- 3 819 539
- US-A- 4 853 476
- JOURNAL OF MEDICINAL CHEMISTRY, vol.36, no.1, 8 Janvier 1993, WASHINGTON US pages 173 - 176 S.R. BERTENSHAW ET AL. 'Phosphorus-Containing Inhibitors of Endothelin Converting Enzyme: Effects of the Electronic Nature of Phosphorus on Inhibitor Potency'
- JOURNAL OF MEDICINAL CHEMISTRY, vol.33, no.1, Janvier 1990, WASHINGTON US pages 263 - 273 Z.P. KORTYLEWICZ AND R.E. GALARDY 'Phosphoramidate Peptide Inhibitors of Human Skin Fibroblast Collagenase'
- TETRAHEDRON LETTERS, vol.1, 1972, OXFORD GB pages 97 - 100 S. UMEZAWA ET AL. 'A New Microbial Metabolite Phosphoramidon (Isolation and Structure)'

## Description

La présente invention concerne de nouveaux dérivés d'acide phosphonique, leur procédé de préparation et les compositions pharmaceutiques qui les contiennent.
Les endothélines sont des peptides de 21 acides aminés ayant des activités vasoconstrictives très puissantes. Ces endothélines sont synthétisées à partir d'un précurseur, la big endothéline, par une enzyme appelée "endothelin converting enzyme" ou pro-endothéline convertase.
Cette enzyme appartient à la classe des métalloprotéases et elle peut être inhibée par le phosphoramidon. Une augmentation des taux plasmatiques de l'endothéline a été démontrée dans des maladies telles que l'hypertension, l'angine, l'infarctus du myocarde, l'insuffisance rénale, le choc, le diabète, l'hypercholestérolémie, le vasospasme cérébral, la maladie de Raynaud, l'arthrite inflammatoire, l'insuffisance cardiaque et l'hypertension pulmonaire. Il a donc été postulé que l'endothéline pourrait jouer un rôle dans l'ischémie périphérique et myocardique, l'hypertension, l'insuffisance rénale, la vasoconstriction pulmonaire hypoxique, l'asthme, l'athérosclérose et l'arthrite. Il était donc particulièrement intéressant de synthétiser des substances inhibitrices de pro-endothéline convertase.

L'état antérieur de la technique est illustré notamment par les brevets EP 0518299 et WO 9201468 et la publication J. Med. Chem., vol. 36, n° 1, 8 Janvier 1993, p. 173-176.

L'invention concerne plus particulièrement les composés de formule (I): 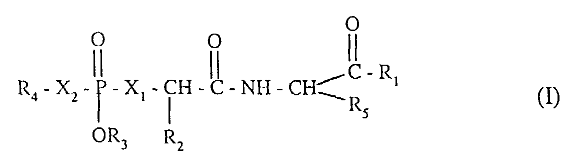 dans laquelle :
- R₁: représente un groupement hydroxy, alkoxy (C₁-C₆) linéaire ou ramifié ou amino (substitué ou non par 1 ou 2 groupements alkyle (C₁-C₆) linéaire ou ramifié),
- R₂: représente un groupement alkyle (C₁-C₆) linéaire ou ramifié substitué ou non par un groupement phényle ou cycloalkyle (C₃-C₇),
- X₁: représente -NH-,
- X₂: représente -O-,
- R₃: représente un atome d'hydrogène, un groupement alkyle (C₁-C₆) linéaire ou ramifié ou phényle,
- R₄: représente un groupement alkyle (C₁-C₆) linéaire ou ramifié toujours substitué par un ou plusieurs groupements, identiques ou différents, hydroxy, benzyloxy, benzyloxycarbonylamino, amino, mono ou dialkylamino (C₁-C₆) linéaire ou ramifié, acétoxy ou 2,2-diméthyl-1,3-dioxolan-4-yl,
- R₅: représente un groupement indol-3-ylméthyle, naphtylméthyle, alkyle (C₁-C₆) linéaire ou ramifié, phényle ou benzyle,
leurs énantiomères, diastéréoisomères et épimères ainsi que leurs sels d'addition à un acide ou à une base pharmaceutiquement acceptable.

Parmi les bases pharmaceutiquement acceptables, on peut citer à titre non limitatif l'hydroxyde de sodium, l'hydroxyde de potassium, l'hydroxyde de lithium, la triéthylamine, la tert-butylamine, etc...

Parmi les acides pharmaceutiquement acceptables, on peut citer à titre non limitatif les acides chlorhydrique, sulfurique, tartrique, maléique, fumarique, méthane sulfonique, camphorique, etc...

L'invention s'étend également au procédé de préparation des composés de formule (I) caractérisé en ce que l'on utilise comme produit de départ l'amino-acide de formule (II), sous forme racémioue ou d'énantiomère pur : 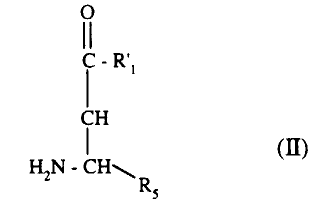 dans laquelle :
- R'₁: représente un groupement alkoxy (C₁-C₆) linéaire ou ramifié ou amino (substitué ou non par 1 ou 2 groupements alkyle (C₁-C₆) linéaire ou ramifié),
et
- R₅: a la même signification que dans la formule (I),
que l'on fait réagir avec un composé de formule (III) sous forme racémique ou d'énantiomère pur : 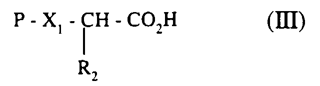 dans laquelle R₂ et X₁ ont la même signification que dans la formule (I) et P représente un groupement protecteur approprié,
pour conduire à un composé de formule (IV), dont on sépare éventuellement les isomères selon une technique classique de séparation : 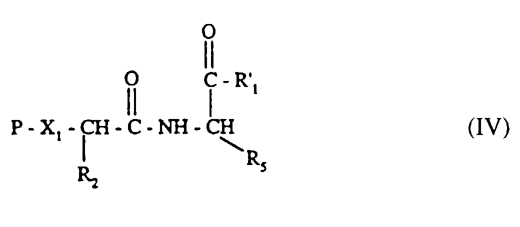 dans laquelle R'₁, R₂, X₁, R₅ et P ont la même signification que précédemment,
que l'on déprotège selon la nature de P par une technique appropriée, pour conduire au composé de formule (V) : 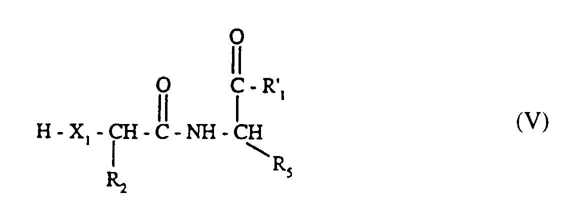 dans laquelle R'₁, R₂ , R₅ et X₁ ont la même signification que précédemment,
que l'on fait réagir :
- en milieu inerte, à température ambiante, avec une solution chloroformique préalablement préparée par agitation en milieu inerte :
- d'un équivalent d'un phényl dichlorophosphate de formule (VII) : 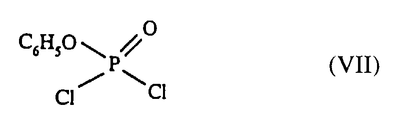
- d'un équivalent du composé de formule (VIII) :

   R'₄ - X₂ - H (VIII)

   dans laquelle
   - X₂: a la même signification que dans la formule (I),
   - R'₄: représente un groupement alkyle (substitué par un ou plusieurs groupements benzyloxy, benzyloxycarbonylamino, acétoxy ou dialkylamino (C₁-C₆) linéaire ou ramifié ou 2,2-diméthyl-1,3-dioxolan-4-yl),
- et, de deux équivalents de triéthylamine,
pour conduire, après déprotection en milieu basique, au composé de formule (I/a), cas particulier des composés de formule (I) : 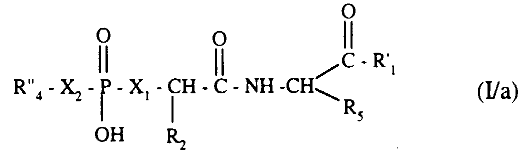 dans laquelle R'₁, R₂, R₅ et X₂ ont la même signification que précédemment et R''₄ représente un groupement alkyle (substitué par un ou plusieurs groupements hydroxy, benzyloxy, benzyloxycarbonylamino, acétoxy ou dialkylamino (C₁-C₆) linéaire ou ramifié ou 2,2-diméthyl-1,3-dioxolan-4-yl),
composé de formule (I/a) dont on peut transformer le groupement R''₄ lorsque celui-ci représente :
- un groupement alkyle substitué par un ou plusieurs groupements benzyloxy en groupement alkyle substitué par un ou plusieurs groupements hydroxy, par hydrogénation catalytique,
- un groupement alkyle substitué par un ou plusieurs groupements acétoxy en groupement alkyle substitué par un ou plusieurs groupements hydroxy, par réaction avec de l'hydroxyde de lithium ou de sodium,
composé de formule (I/a) :
- dont on peut transformer le groupement R'₁ lorsque celui-ci représente un groupement alkoxy en groupement hydroxy correspondant,
- que l'on peut purifier selon des techniques classiques de purification,
- dont on peut séparer les isomères selon des techniques classiques de séparation,
- et, que l'on peut transformer en sel d'acide ou de base correspondant.

Le procédé de préparation des composés de formule (I) s'étend également à la synthèse du composé de référence, le phosphoramidon de formule :

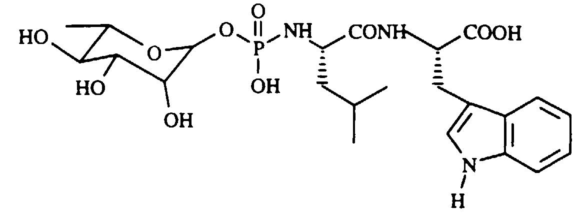

Le phosphoramidon était jusqu'alors isolé de souches de Streptomyces tanashiensis (Tet. Lett. 43, 97-100, 1972).

La présente invention a également pour objet les compositions pharmaceutiques renfermant comme principe actif au moins un composé de formule générale (I) ou un de ses sels d'addition à un acide pharmacologiquement acceptable, seul ou en combinaison avec un ou plusieurs excipients ou véhicules inertes, non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie varie selon l'âge et le poids du patient, la nature et la sévérité de l'affection ainsi que la voie d'administration.

Celle-ci peut être orale, nasale, rectale ou parentérale. D'une manière générale, la posologie unitaire s'échelonne entre 0,1 et 1000 mg pour un traitement en 1 à 3 prises par 24 heures.

Les exemples suivants illustrent l'invention mais ne la limitent an aucune façon. Les produits de départ utilisés dans les exemples sont des produits de départ connus ou préparés selon des modes opératoires connus.

Les abréviations utilisées dans les exemples sont les suivantes :
- Leu: à la place du résidu leucine
- Trp: à la place du résidu tryptophane
- Et: à la place d'éthyle
- Val: à la place du résidu valine
- Ile: à la place du résidu isoleucine
- cyclohexylAla: à la place du résidu cyclohexylalanine
- tert-butylGly: à la place du résidu tert-butylglycine
- Phe: à la place du résidu phénylalanine
- Nal: à la place du résidu 2-naphtylalanine

### Exemple 1 : N-[(2,3-Dihydroxypropoxy)hydroxyphosphinyl]-(S)Leu-(S)Trp-OH, disel de sodium

### Stade A: N-[(2,3-Diacétoxypropoxy)phénoxyphosphinyl]-(S)Leu-(S)Trp-OEt

4,8 mmoles de 2,3-diacétoxypropanol en solution dans du chloroforme anhydre sont ajoutées à 0°-5°C à un mélange contenant 4,8 mmoles de phényldichlorophosphate et 9,6 mmoles de triéthylamine dans le chloroforme anhydre. L'ensemble est agité 3 heures à température ambiante. Une solution contenant 4,8 mmoles de (S)Leu-(S)-Trp-OEt dans le chloroforme anhydre (préparée par couplage peptidique selon la technique décrite par W. KONIG et R. GEIGER (Ber, 103, 788, 1970) de Z-Leu-OH et de H-Trp-OEt) est alors ajoutée au mélange précédent. Après 48 heures d'agitation à température ambiante, le mélange réactionnel est lavé à l'eau, puis avec une solution saturée de chlorure de sodium, séché, filtré et évaporé sous vide. L'huile obtenue est purifiée par chromatographie sur colonne de silice en utilisant un mélange dichlorométhane/acétate d'éthyle (70/30) et conduit au produit attendu.

### Stade B : N-[(2,3-Dihydroxypropoxy)hydroxyphosphinyl]-(S)Leu-(S)Trp-OH, disel de sodium

Le produit obtenu au stade A est saponifié dans un mélange contenant 1,75 ml de soude 1N et 20 ml d'éthanol refroidi à 0°-5°C. Après évaporation de l'éthanol, l'huile résiduelle est diluée à l'eau, lavée plusieurs fois au dichlorométhane puis lyophilisée. Le lyophilisat est purifié par passage sur colonne SEPHADEX (LH20). Les phases aqueuses réunies sont à nouveau lyophilisées.
Rendement : 50 %
Spectre de masse : FAB : [M+Na]⁺ : m/z = 538

### Exemple 2 : N-[(2-Benzyloxyéthoxy)hydroxyphosphinyl]-(S)Leu-(S)Trp-OH, disel de sodium

Le produit attendu est obtenu selon le procédé décrit dans l'exemple 1 en remplaçant au stade A le 2,3-diacétoxypropanol par le 2-benzyloxyéthanol.
Spectre de masse : FAB : [M+H]⁺ : m/z = 576

### Exemple 3 : N-[(2-Hydroxyéthoxy)hydroxyphosphinyl]-(S)Leu-(S)Trp-OEt, sel de sodium

Le produit attendu est obtenu par hydrogénation catalytique de 2,7 mmoles du composé obtenu au stade A de l'exemple 2, à pression atmosphérique et à température ambiante, en présence de 50 ml d'éthanol, de 230 mg de bicarbonate de sodium et de 6 ml d'eau et d'un mélange (50/50) PtO₂ - Pd/C_{10%} comme catalyseurs. Après 48 heures d'hydrogénation, les catalyseurs sont filtrés et la solution évaporée. L'huile résiduelle est reprise à l'eau, lavée au dichlorométhane et lyophilisée. Le lyophilisat est alors dissous dans l'eau, purifié sur colonne SEPHADEX (LH20) puis à nouveau lyophilisé et conduit au produit attendu.
Spectre de masse : FAB : [M+H]⁺ : m/z = 492

### Exemple 4 : N-[(2-Hydroxyéthoxy)hydroxyphosphinyl]-(S)Leu-(S)Trp-OH, disel de sodium

Le produit attendu est obtenu par hydrogénation catalytique du composé décrit dans l'exemple 2 en utilisant la méthode décrite dans l'exemple 3.
Spectre de masse : FAB : [M+H]⁺ : m/z = 486

### Exemple 5 : N-[(3-Hydroxypropoxy)hydroxyphosphinyl]-(S)Leu-(S)Trp-OH, disel de lithium

Le produit attendu est obtenu selon le procédé décrit dans l'exemple 1 en remplaçant au stade A le 2,3-diacétoxypropanol par le 3-acétoxypropanol et par saponification à l'aide d'hydroxyde de lithium.
Spectre de masse : FAB : [M+H]⁺ : m/z = 468

### Exemple 6: N-[[2-(Benzyloxycarbonylamino)éthoxy]hydroxyphosphinyl]-(S)Leu-(S)Trp-OEt, sel de lithium

Le produit attendu est obtenu selon le procédé décrit dans l'exemple 1 en remplaçant au stade A le 2,3-diacétoxypropanol par le 2-(benzyloxycarbonylamino) éthanol. La saponification est réalisée à l'aide d'hydroxyde de lithium.
Spectre de masse : FAB : [M+H]⁺ : m/z = 609

### Exemple 7 : N-{[(2,2-Diméthyl-1,3-dioxolan-4-yl)méthoxy]hydroxyphosphinyl}-(S)Leu-(S)Trp-OEt, sel de sodium

### Stade A : N-{[(2,2-Diméthyl-1,3-dioxolan-4-yl)méthoxy]phénoxyphosphinyl}-(S)Leu-(S)Trp-OEt

Le produit attendu est obtenu selon le procédé décrit au stade A de l'exemple 1 en remplaçant le 2,3-diacétoxypropanol par le 2,2-diméthyl-1,3-dioxolan-4-yl-méthanol.

### Stade B: N-{[(2,2-Diméthyl-1,3-dioxolan-4-yl)méthoxy]hydroxyphosphinyl}-(S)Leu-(S)Trp-OEt, sel de sodium

A une solution contenant 63 mmoles du composé obtenu au stade précédent dans 30 ml d'éthanol est ajoutée une solution contenant 63 mmoles de bicarbonate de sodium dans 5 ml d'eau. Le milieu est hydrogéné 48 heures à température ambiante en présence d'oxyde de platine comme catalyseur sous une pression de 1200 mbars. Après filtration du catalyseur et évaporation du solvant, le résidu est repris par 50 ml d'eau. Après lavage de la phase aqueuse à l'acétate d'éthyle et filtration, le produit attendu est obtenu après lyophilisation.
Spectre de masse : FAB : [M+H]⁺ : m/z = 562
- Infrarouge (nujol) :: δ_{co} ester = 1728 cm⁻¹
δ_{co} amides = 1653 cm⁻¹

### Exemple 8 : N-{[(1-Hydroxyméthyl-2-hydroxy)éthoxy]hydroxyphosphinyl}-(S)Leu-(S)Trp-OH, disel de lithium

Le produit attendu est obtenu selon le procédé décrit dans l'exemple 5 en remplaçant au stade A le 3-acétoxypropanol par le 1,3-diacétoxypropanol-2.
Spectre de masse : FAB : [M+H]⁺ : m/z = 484

### Exemple 9 : N-[(3-Aminopropoxy)hydroxyphosphinyl]-(S)Leu-(S)Trp-OEt, sel de lithium

### Stades A et B : N-{[(3-Benzyloxycarbonylamino)propoxy]hydroxyphosphinyl}-(S)Leu-(S)Trp-OEt, sel de lithium

Le produit attendu est obtenu selon le procédé décrit dans l'exemple 6 en remplaçant au stade A le (benzyloxycarbonylamino)éthanol par le (benzyloxycarbonylamino)propanol.

### Stade C : N-[(3-Aminopropoxy)hydroxyphosphinyl]-(S)Leu-(S)Trp-OEt, sel de lithium

Le produit attendu est obtenu après hydrogénation catalytique du composé décrit au stade précédent en utilisant le palladium/charbon comme catalyseur, à température ambiante, sous une pression de 1200 mbars, puis lyophilisation.
Spectre de masse : FAB : [M+2H-Li]⁺ : m/z = 483

### Exemple 10 : N-[((R)-2,3-Dihydroxypropoxy)hydroxyphosphinyl]-(S)Leu-(S)Trp-OH, disel de lithium

Le produit attendu est obtenu selon le procédé décrit dans l'exemple 1 en remplaçant au stade A le 2,3-diacétoxypropanol par l'isomère (R)-2,3-diacétoxypropanol et par saponification à l'aide d'hydroxyde de lithium.
Spectre de masse : FAB : [M+H]⁺: m/z = 484

### Exemple 11 : N-[((S)-2,3-Dihydroxypropoxy)hydroxyphosphinyl]-(S)Leu-(S)Trp-OH, disel de lithium

Le produit attendu est obtenu selon le procédé décrit dans l'exemple 1 en remplaçant au stade A le 2,3-diacétoxypropanol par l'isomère (S)-2,3-diacétoxypropanol et par saponification à l'aide d'hydroxyde de lithium.
Spectre de masse : FAB : [M+H]⁺: m/z = 484

### Exemple 12 : N-[(3,4-Dihydroxybutoxy)hydroxyphosphinyl]-(S)Leu-(S)Trp-OH, disel de lithium

Le produit attendu est obtenu selon le procédé décrit dans l'exemple 1 en remplaçant au stade A le 2,3-diacétoxypropanol par le 3,4-diacétoxybutanol et par saponification à l'aide d'hydroxyde de lithium.
Spectre de masse : FAB: [M+H]⁺: m/z = 498

### Exemple 13 : N-(1-Hydroxyméthyl-3-hydroxypropoxy)hydroxyphosphinyl-(S)Leu-(S)Trp-OH, disel de lithium

Le produit attendu est obtenu selon le procédé décrit dans l'exemple 1 en remplaçant au stade A le 2,3-diacétoxypropanol par le 1-acétoxyméthyl-3-acétoxypropanol et par saponification à l'aide d'hydroxyde de lithium.
Spectre de masse : FAB : [M+H]⁺: m/z = 498

### Exemple 14 : N-(2,4-Hydroxybutoxy)hydroxyphosphinyl-(S)Leu-(S)Trp-OH, disel de lithium

Le produit attendu est obtenu selon le procédé décrit dans l'exemple 1 en remplaçant au stade A le 2,3-diacétoxypropanol par le 2,4-diacétoxybutanol et par saponification à l'aide d'hydroxyde de lithium.
Spectre de masse : FAB : [M+H]⁺: m/z = 498

### Exemple 15 : N-[(2,3,4-Trihydroxybutoxy)hydroxyphosphinyl]-(S)Leu-(S)Trp-OH, disel de lithium

Le produit attendu est obtenu selon le procédé décrit dans l'exemple 1 en remplaçant au stade A le 2,3-diacétoxypropanol par le 2,3,4-triacétoxybutanol et par saponification à l'aide d'hydroxyde de lithium.
Spectre de masse : FAB : [M+H]⁺: m/z = 514

### Exemple 16: N-[(2,3-Dihydroxypropoxy)hydroxyphosphinyl]-(S)Leu-(S)Trp-OEt, sel de sodium

Le produit attendu est obtenu selon le procédé décrit dans l'exemple 7 en utilisant au stade A le 2,3-diacétoxypropanol.

### Exemple 17: N-[(2,3-Dihydroxypropoxy)hydroxyphosphinyl]-(S)Val-(S)Trp-OH, disel de lithium

Le produit attendu est obtenu selon le procédé décrit dans l'exemple 1 en remplaçant au stade A le (S)Leu-(S)Trp-OEt par le (S)Val-(S)Trp-OEt et par saponification à l'aide d'hydroxyde de lithium.
Spectre de masse : FAB : [M+H]⁺: m/z = 470

### Exemple 18: N-[(2,3-Dihydroxypropoxy)hydroxyphosphinyl]-(S)Ile-(S)Trp-OH, disel de lithium

Le produit attendu est obtenu selon le procédé décrit dans l'exemple 1 en remplaçant au stade A le (S)Leu-(S)Trp-OEt par le (S)Ile-(S)Trp-OEt et par saponification à l'aide d'hydroxyde de lithium.
Spectre de masse : FAB : [M+H]⁺: m/z = 484

### Exemple 19: N-[(2,3-Dihydroxypropoxy)hydroxyphosphinyl]-(S)cyclohexylAla-(S)Trp-OH, disel de lithium

Le produit attendu est obtenu selon le procédé décrit dans l'exemple 1 en remplaçant au stade A le (S)Leu-(S)Trp-OEt par le (S)cyclohexylAla-(S)Trp-OEt et par saponification à l'aide d'hydroxyde de lithium.
Spectre de masse : FAB : [M+H]⁺: m/z = 524

### Exemple 20: N-[(2,3-Dihydroxypropoxy)hydroxyphosphinyl]-(S)tert-butylGly-(S)Trp-OH, disel de lithium

Le produit attendu est obtenu selon le procédé décrit dans l'exemple 1 en remplaçant au stade A le (S)Leu-(S)Trp-OEt par le (S)tert-butylGly-(S)Trp-OEt et par saponification à l'aide d'hydroxyde de lithium.
Spectre de masse : FAB : [M+H]⁺: m/z = 484

### Exemple 21: N-[(2,3-Dihydroxypropoxy)hydroxyphosphinyl]-(S)Leu-(S)tert-butylGly-OH, disel de lithium

Le produit attendu est obtenu selon le procédé décrit dans l'exemple 1 en remplaçant au stade A le (S)Leu-(S)Trp-OEt par le (S)Leu-(S)tert-butylGly-OEt et par saponification à l'aide d'hydroxyde de lithium.
Spectre de masse : FAB : [M+H]⁺: m/z = 411

### Exemple 22: N-[(2,3-Dihydroxypropoxy)hydroxyphosphinyl]-(S)Leu-(S)Phe-OH, disel de lithium

Le produit attendu est obtenu selon le procédé décrit dans l'exemple 1 en remplaçant au stade A le (S)Leu-(S)Trp-OEt par le (S)Leu-(S)Phe-OEt et par saponification à l'aide d'hydroxyde de lithium.
Spectre de masse : FAB : [M+H]⁺: m/z = 445

### Exemple 23: N-[(2,3-Dihydroxypropoxy)hydroxyphosphinyl]-(S)Leu-(S)Nal-OEt, sel de sodium

Le produit attendu est obtenu selon le procédé décrit dans l'exemple 1 en remplaçant au stade A le (S)Leu-(S)Trp-OEt par le (S)Leu-(S)Nal-OEt et par saponification à l'aide d'hydroxyde de lithium.
Spectre de masse : FAB : [M+H]⁺: m/z = 495

### Exemple 24: Phosphoramidon, disel de sodium (N-[α-(S)(rhamnopyranosyloxy)hydroxyphosphinyl]-(S)Leu-(S)Trp-OH, disel de sodium)

Le produit attendu est obtenu selon le procédé décrit dans l'exemple 1 en remplaçant au stade A le 2,3-diacétoxypropanol par le triacétate de rhamnose. Les isomères obtenus sont alors séparés et purifiés par chromatographie sur colonne de silice phase inverse (C₁₈) en utlisant l'eau comme éluant. Le produit attendu présente les mêmes caractéristiques physicochimiques que celle du phosphoramidon commercial.
Spectre de masse : FAB : [M+H]⁺: m/z = 588
Pouvoir rotatoire : α_{D}²⁰ = -30,1 (c = 0,96 %, eau)

### Etude pharmacologique des composés de l'invention

### Exemple 25: Etude in vivo des composés de l'invention sur le rat spinalisé

Des rats Sprague-Dawley (300-400 g) sont anesthésiés avec de l'éther. Ensuite, les animaux sont spinalisés et placés sous respiration artificielle. Les nerfs vagues sont sectionnés et les artères carotides ligaturées. Un cathéter est introduit dans une des artères carotides pour la mesure de la pression artérielle. Un deuxième cathéter est introduit dans la veine du pénis pour permettre l'injection des substances.
Après une période de stabilisation, les animaux reçoivent une injection d'endothéline-1 (ET-1 ; 0,5 nmol/kg) ou de son précurseur, la big endothéline-1 (big ET-1 ; 1 nmol/kg). Les réponses pressives sont enregistrées et après retour à la pression initiale (1 h 30 à 2 heures) une deuxième injection de l'ET-1 ou de la big ET-1 est donnée en présence ou en absence d'une infusion d'un produit de l'invention ou de la substance de référence, le phosphoramidon. Le phosphoramidon et les produits de l'invention n'ont pas influencé les hypertensions produites par l'ET-1. Par contre, ils ont inhibé de façon dose-dépendante les réponses pressives induites par la big ET-1 indiquant une inhibition de l'ECE.
Les résultats des IC₅₀ de ces substances vis-à-vis de la big ET-1 sont rassemblés ci-dessous.

| Exemple | IC₅₀ (µg/kg/min) |
|---|---|
| 1 | 100 |
| 3 | 280 |
| 4 | 430 |
| 8 | 480 |
| 9 | 200 |
| 10 | 230 |
| 11 | 100 |
| 12 | 140 |
| Phosphoramidon | 125 |

### Exemple 26: Etude in vitro des composés de l'invention sur le rein isolé perfusé de rat

Les études sont effectuées sur des reins préparés chez des rats Wistar mâles (300-400 g). Les rats sont anesthésiés avec du sodium pentobarbitone (50 mg/kg i.p.) et le rein gauche est préparé afin de permettre la perfusion avec de la solution Tyrode. Les variations de la pression de perfusion sont mesurées de façon continue. Le débit de perfusion est de 6 ml/min. Après stabilisation, une injection en bolus d'ET-1 (0,03 nmol) ou de big ET-1 (0,4 nmol) est effectuée et la réponse pressive est enregistrée. Après retour à la pression basale, une seconde injection d'ET-1 ou de big ET-1 est faite, soit en solution contrôle, soit en présence du phosphoramidon ou d'un des produits de l'invention. Aucun des produits n'a influencé les réponses pressives à l'ET-1. Par contre, le phosphoramidon et les composés de l'invention ont inhibé de façon importante les réponses pressives obtenues avec la big ET-1. Les IC₅₀ des produits sont calculés et les résultats sont donnés en µM.

| Exemple | IC₅₀ (µM) |
|---|---|
| 1 | 4 |
| 3 | 5 |
| 4 | 7 |
| Phosphoramidon | 0,9 |

### Exemple 27 : Composition pharmaceutique

### Formule de composition pour 1000 comprimés dosés à 10 mg

| Formule de composition pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 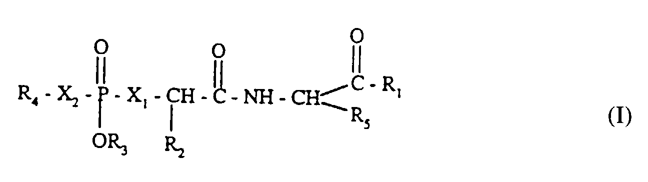 dans laquelle :
R₁ représente un groupement hydroxy, alkoxy (C₁-C₆) linéaire ou ramifié ou amino (substitué ou non par 1 ou 2 groupements alkyle (C₁-C₆) linéaire ou ramifié),
R₂ représente un groupement alkyle (C₁-C₆) linéaire ou ramifié substitué ou non par un groupement phényle ou cycloalkyle (C₃-C₇),
X₁ représente -NH-,
X₂ représente -O-,
R₃ représente un atome d'hydrogène, un groupement alkyle (C₁-C₆) linéaire ou ramifié ou phényle,
R₄ représente un groupement alkyle (C₁-C₆) linéaire ou ramifié toujours substitué par un ou plusieurs groupements, identiques ou différents, hydroxy, benzyloxy,benzyloxycarbonylamino, amino, mono ou dialkylamino (C₁-C₆) linéaire ou ramifié, acétoxy ou 2,2-diméthyl-1,3-dioxolan-4-yl,
R₅ représente un groupement indol-3-ylméthyle, naphtylméthyle, alkyle (C₁-C₆) linéaire ou ramifié, phényle ou benzyle,
leurs énantiomères, diastéréoisomères et épimères ainsi que leurs sels d'addition à un acide ou à une base pharmaceutiquement acceptable.

2. Composés de formule (I) selon la revendication 1 tels que R₄ représente un groupement alkyle (C₁-C₆) toujours substitué par un ou plusieurs groupements hydroxy, leurs énantiomères, diastéréoisomères et épimères ainsi que leurs sels d'addition à un acide ou à une base pharmaceutiquement acceptable.

3. Composés de formule (I) selon la revendication 1 tels que R₅ représente un groupement indol-3-yl méthyle, leurs énantiomères, diastéréoisomères et épimères ainsi que leurs sels d'addition à un acide ou une base pharmaceutiquement acceptable.

4. Composé de formule (I) selon la revendication 1 qui est le N-[(2,3-Dihydroxypropoxy)hydroxyphosphinyl]-(S)Leu-(S)-Trp-OH, ses isomères ainsi que ses sels d'addition à un acide ou une base pharmaceutiquement acceptable.

5. Procédé de préparation des composés de formule (I) selon la revendication 1 caractérisé en ce que l'on utilise comme produit de départ l'amino-acide de formule (II), sous forme racémique ou d'énantiomère pur : 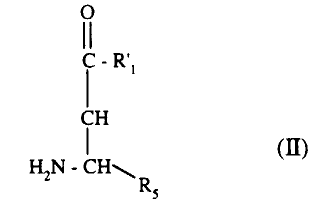 dans laquelle :
R'₁ représente un groupement alkoxy (C₁-C₆) linéaire ou ramifié ou amino (substitué ou non par 1 ou 2 groupements alkyle (C₁-C₆) linéaire ou ramifié), et
R₅ a la même signification que dans la formule (I),
que l'on fait réagir avec un composé de formule (III) sous forme racémique ou d'énantiomère pur : 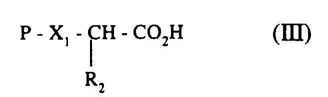 dans laquelle R₂ et X₁ ont la même signification que dans la formule (I) et P représente un groupement protecteur approprié,
pour conduire à un composé de formule (IV), dont on sépare éventuellement les isomères selon une technique classique de séparation : 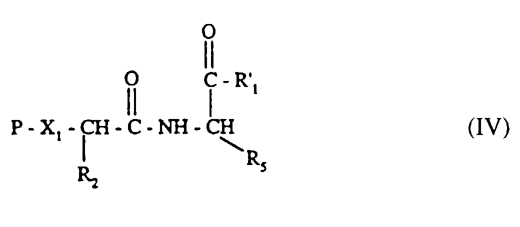 dans laquelle R'₁, R₂, X₁, R₅ et P ont la même signification que précédemment,
que l'on déprotège selon la nature de P par une technique appropriée, pour conduire au composé de formule (V) : 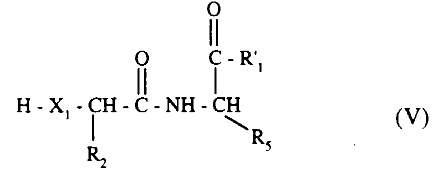 dans laquelle R'₁, R₂, R₅ et X₁ ont la même signification que précédemment,
que l'on fait réagir :
- en milieu inerte, à température ambiante, avec une solution chloroformique préalablement préparée par agitation en milieu inerte :
- d'un équivalent d'un phényl dichlorophosphate de formule (VII) : 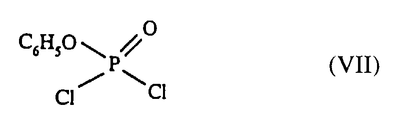
- d'un équivalent du composé de formule (VIII) :
R'₄ - X₂ - H (VIII)
dans laquelle
X₂ a la même signification que dans la formule (I),
R'₄ représente un groupement alkyle (substitué par un ou plusieurs groupements benzyloxy, benzyloxycarbonylamino, acétoxy ou dialkylamino (C₁-C₆) linéaire ou ramifié ou 2,2-diméthyl-1,3-dioxolan-4-yl),
- et, de deux équivalents de triéthylamine,
pour conduire, après déprotection en milieu basique, au composé de formule (I/a), cas particulier des composés de formule (I) : 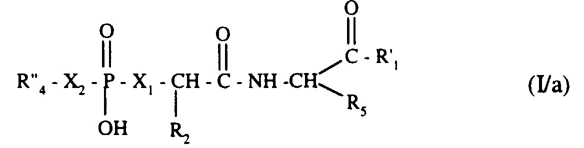 dans laquelle R'₁, R₂, R₅ et X₂ ont la même signification que précédemment et R''₄ représente un groupement alkyle (substitué par un ou plusieurs groupements hydroxy, benzyloxy, benzyloxycarbonylamino, acétoxy ou dialkylamino (C₁-C₆) linéaire ou ramifié ou 2,2-diméthyl-1,3-dioxolan-4-yl),
composé de formule (I/a) dont on peut transformer le groupement R''₄ lorsque celui-ci représente :
- un groupement alkyle substitué par un ou plusieurs groupements benzyloxy en groupement alkyle substitué par un ou plusieurs groupements hydroxy, par hydrogénation catalytique,
- un groupement alkyle substitué par un ou plusieurs groupements acétoxy en groupement alkyle substitué par un ou plusieurs groupements hydroxy, par réaction avec de l'hydroxyde de lithium ou de sodium,
composé de formule (I/a) :
- dont on peut transformer le groupement R'₁ lorsque celui-ci représente un groupement alkoxy en groupement hydroxy correspondant,
- que l'on peut purifier selon des techniques classiques de purification,
- dont on peut séparer les isomères selon des techniques classiques de séparation,
- et, que l'on peut transformer en sel d'acide ou de base correspondant.

6. Procédé de préparation du phosphoramidon de formule : 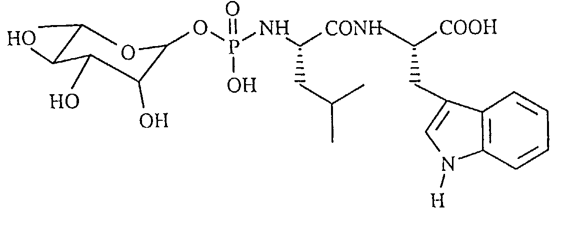 caractérisé en ce qu'il est identique à celui décrit dans la revendication 5 en remplacant le composé de formule (VIII) par le composé de formule :

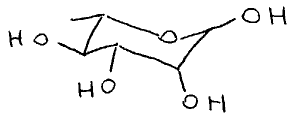

7. Composition pharmaceutique contenant comme principe actif un composé selon l'une quelconque des revendications 1 à 4, seul ou en combinaison avec un ou plusieurs excipients ou véhicules inertes, non toxiques, pharmaceutiquement acceptables.

8. Composition pharmaceutique selon la revendication 7 contenant un principe actif selon l'une quelconque des revendicatons 1 à 4 utiles en tant qu'inhibiteur d'endotheline convertase.

## Claims

1. Compounds of formula (I): 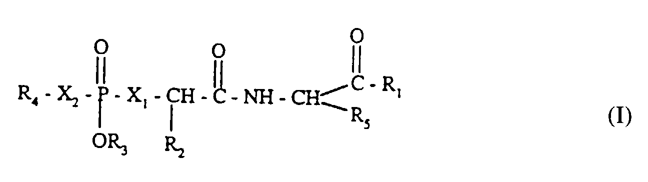 wherein:
R₁ represents a hydroxy group, a linear or branched (C₁-C₆)alkoxy group or an amino group (unsubstituted or substituted by 1 or 2 linear or branched (C₁-C₆)alkyl groups),
R₂ represents a linear or branched (C₁-C₆)alkyl group unsubstituted or substituted by a phenyl or (C₃-C₇)cycloalkyl group,
X₁ represents -NH-,
X₂ represents -O-,
R₃ represents a hydrogen atom, a linear or branched (C₁-C₆)alkyl group or a phenyl group,
R₄ represents a linear or branched (C₁-C₆)alkyl group always substituted by one or more identical or different groups: hydroxy, benzyloxy, benzyloxycarbonylamino, amino, mono- or di-(C₁-C₆)alkylamino in which the(each) alkyl moiety is linear or branched, acetoxy, 2,2-dimethyl-1,3-dioxolan-4-yl,
R₅ represents an indol-3-ylmethyl, naphthylmethyl, linear or branched (C₁-C₆)alkyl, phenyl or benzyl group,
their enantiomers, diastereoisomers and epimers and addition salts thereof with a pharmaceutically acceptable acid or base.

2. Compounds of formula (I) according to claim 1 wherein R₄ represents a (C₁-C₆)alkyl group always substituted by one or more hydroxy groups, their enantiomers, diastereoisomers and epimers and addition salts thereof with a pharmaceutically acceptable acid or base.

3. Compounds of formula (I) according to claim 1, wherein R₅ represents an indol-3-ylmethyl group, their enantiomers, diastereoisomers and epimers and addition salts thereof with a pharmaceutically acceptable acid or base.

4. Compound of formula (I) according to claim 1 which is N-[(2,3-dihydroxypropoxy)hydroxyphosphinyl]-(S)Leu-(S)Trp-OH, its isomers and addition salts thereof with a pharmaceutically acceptable acid or base.

5. Process for the preparation of compounds of formula (I) according to claim 1, characterised in that there is used as starting material the amino acid of formula (II), in racemic form or in the form of a pure enantiomer: 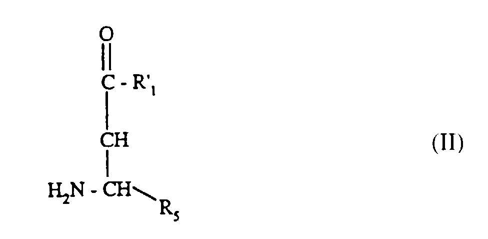 wherein:
R'₁ represents a linear or branched (C₁-C₆)alkoxy group or an amino group (unsubstituted or substituted by 1 or 2 linear or branched (C₁-C₆)alkyl groups),
and
R₅ is as defined for formula (I),
which is reacted with a compound of formula (III) in racemic form or in the form of a pure enantiomer: 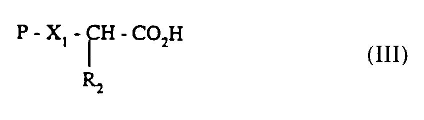 wherein R₂ and X₁ are as defined for formula (I) and P represents a suitable protecting group,
to yield a compound of formula (IV), which is separated into its isomers, where appropriate, according to a conventional separation technique: 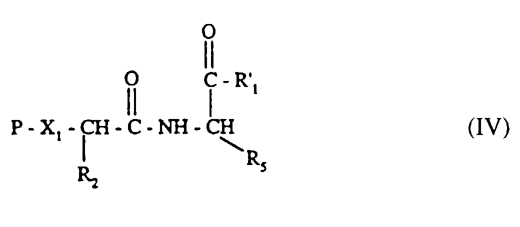 wherein R'₁, R₂, X₁, R₅ and P are as defined hereinbefore,
which is deprotected, depending upon the nature of P, using a suitable technique, to yield a compound of formula (V): 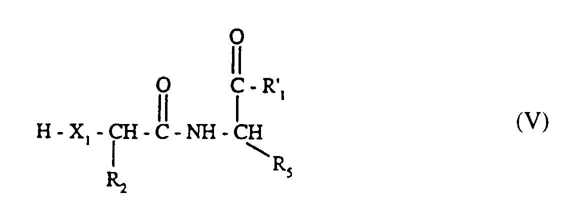 wherein R'₁, R₂, R₅ and X₁ are as defined hereinbefore,
which is reacted:
- in an inert medium, at room temperature, with a chloroformic solution prepared in advance by stirring the following in an inert medium:
- one equivalent of a phenyl dichlorophosphate of formula (VII): 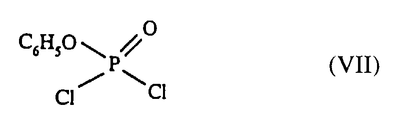
- one equivalent of a compound of formula (VIII):
R'₄ - X₂ - H (VIII)
wherein
X₂ is as defined for formula (I),
R'₄ represents an alkyl group (substituted by one or more groups: benzyloxy, benzyloxycarhonylamino, acetoxy or di-(C₁-C₆)alkylamino in which each alkyl moiety is linear or branched or 2,2-dimethyl-1,3-dioxolan-4-yl), and
- two equivalents of triethylamine,
to yield, after deprotection in a basic medium, a compound of formula (I/a), a particular case of the compounds of formula (I): 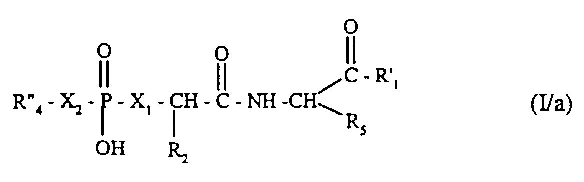 wherein R'₁, R₂, R₅ and X₂ are as defined hereinbefore and R"₄ represents an alkyl group (substituted by one or more groups: hydroxy, benzyloxy, benzyloxycarbonylamino,
acetoxy or di-(C₁-C₆)alkylamino in which each alkyl moiety is linear or branched or 2,2-dimethyl-1,3-dioxolan-4-yl),
in which compound of formula (I/a) the group R"₄ may be converted :
- when that group represents an alkyl group substituted by one or more benzyloxy groups, into an alkyl group substituted by one or more hydroxy groups, by catalytic hydrogenation,
- when that group represents an alkyl group substituted by one or more acetoxy groups, into an alkyl group substituted by one or more hydroxy groups, by reaction with lithium hydroxide or sodium hydroxide,
in which compound of formula (I/a):
- the R'₁ group, when that group represents an alkoxy group, may be converted into the corresponding hydroxy group,
which compound of formula (I/a):
- may be purified according to conventional purification techniques,
- may be separated into its isomers according to conventional separation techniques, and
- may be converted into a corresponding salt of an acid or base.

6. Process for the preparation of the phosphoramidon of formula: 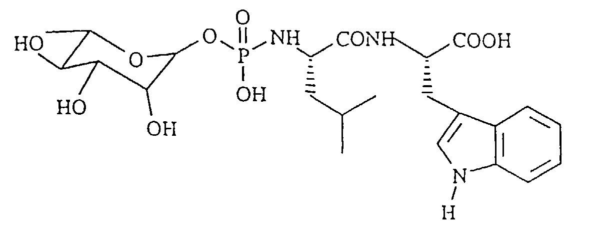 characterised in that it is identical to that described in claim 5, but replacing the compound of formula (VIII) by the compound of formula:

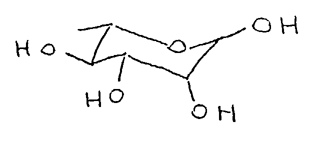

7. Pharmaceutical composition comprising as active ingredient a compound according to any one of claims 1 to 4, alone or in combination with one or more inert, non-toxic, pharmaceutically acceptable excipients or carriers.

8. Pharmaceutical composition according to claim 7 comprising an active ingredient according to any one of claims 1 to 4 for use as an endothelin convertase inhibitor.

## Patentansprüche

1. Verbindungen der Formel (I): 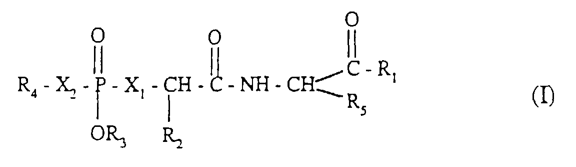 in der:
R₁ eine Hydroxygruppe, eine geradkettige oder verzweigte (C₁-C₆)-Alkoxygruppe oder eine (gegebenenfalls durch 1 oder 2 geradkettige oder verzweigte (C₁-C₆)-Alkylgruppen substituierte) Aminogruppe,
R₂ eine geradkettige oder verzweigte, gegebenenfalls durch eine Phenylgruppe oder (C₃-C₇)-Cycloalkylgruppe substituierte (C₁-C₆)-Alkylgruppe,
X₁ -NH-,
X₂ -O-,
R₃ ein Wasserstoffatom, eine geradkettige oder verzweigte (C₁-C₆)-Alkylgruppe oder eine Phenylgruppe,
R₄ eine geradkettige oder verzweigte, stets durch eine oder mehrere gleichartige oder verschiedene Hydroxy-, Benzyloxy-, Benzyloxycarbonylamino-, Amino-, geradkettige oder verzweigte Mono- oder Di-(C₁-C₆)-amino-, Acetoxy- oder 2,2-Dimethyl-1,3-dioxolan-4-yl-gruppen substituierte (C₁-C₆)-Alkylgruppe,
R₅ eine Indol-3-yl-methyl-, Naphthylmethyl-, geradkettige oder verzweigte (C₁-C₆)-Alkyl-, Phenyl- oder Benzyl-gruppe bedeuten,
deren Enantiomere, Diastereoisomere und Epimere sowie deren Additionssalze mit einer pharmazeutisch annehmbaren Säure oder Base.

2. Verbindungen der Formel (I) nach Anspruch 1, worin R₄ eine (C₁-C₆)-Alkylgruppe darstellt, die stets durch eine oder mehrere Hydroxygruppen substituiert ist, deren Enantiomere, Diastereoisomere und Epimere sowie deren Additionssalze mit einer pharmazeutisch annehmbaren Säure oder Base.

3. Verbindungen der Formel (I) nach Anspruch 1, worin R₅ eine Indol-3-yl-methylgruppe darstellt, sowie deren Enantiomere, Diastereoisomere und Epimere sowie deren Additionssalze mit einer pharmazeutisch annehmbaren Säure oder Base.

4. Verbindungen der Formel (I) nach Anspruch 1, nämlich N-[(2,3-Dihydroxypropoxy)-hydroxyphosphinyl]-(S)Leu-(S)-Trp-OH, deren Isomeren sowie deren Additionssalze mit einer pharmazeutisch annehmbaren Säure oder Base.

5. Verfahren zur Herstellung der Verbindungen der Formel (I) nach Anspruch 1, dadurch gekennzeichnet, daß man als Ausgangsprodukt eine Aminosäure der Formel (II) in Form des Racemats oder des reinen Enantiomeren verwendet: 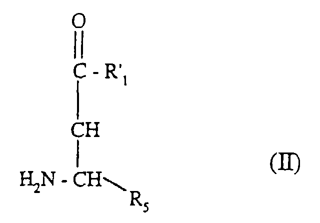 in der:
R'₁ eine geradkettige oder verzweigte (C₁-C₆)-Alkoxygruppe oder eine (gegebenenfalls durch 1 oder 2 geradkettige oder verzweigte (C₁-C₆)-Alkylgruppen substituierte) Aminogruppe bedeutet, und
R₅ die bezüglich der Formel (I) angegebenen Bedeutungen besitzt,
welche man mit einer Verbindung der Formel (III) in Form des Racemats oder des reinen Isomeren umsetzt: 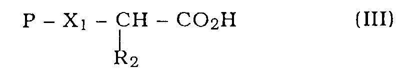 in der R₂ und X₁ die bezüglich der Formel (I) angegebenen Bedeutungen besitzen und P eine geeignete Schutzgruppe darstellt,
zur Bildung einer Verbindung der Formel (IV), die man gegebenenfalls mit Hilfe einer klassischen Trennmethode in die Isomeren auftrennt: 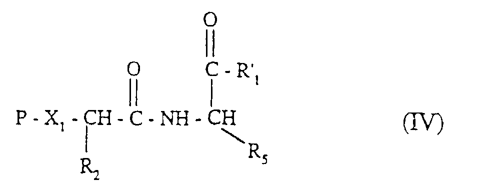 in der R'₁, R₂, X₁, R₅ und P die oben angegebenen Bedeutungen besitzen,
von welcher man in Abhängigkeit von der Art von P mit Hilfe einer geeigneten Technik die Schutzgruppe abspaltet zur Bildung der Verbindung der Formel (V): 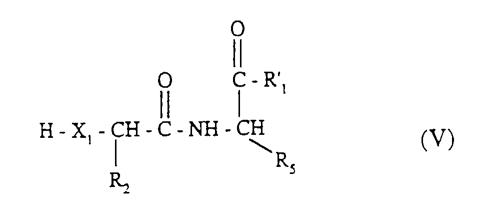 in der R'₁, R₂, R₅ und X₁ die oben angegebenen Bedeutungen besitzen, welche man:
- in inertem Medium bei Raumtemperatur mit einer zuvor hergestellten Chloroformlösung unter Rühren in inertem Medium:
- mit einem Äquivalent eines Phenyldichlorphosphats der Formel (VII): 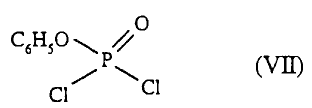
- und einem Äquivalent der Verbindung der Formel (VIII):
R'₄ - X₂ - H (VIII)
in der
X₂ die bezüglich der Formel (I) angegebenen Bedeutungen besitzt und
R'₄ eine (durch eine oder mehrere Benzyloxy-, Benzyloxycarbonylamino-, Acetoxy- oder geradkettige oder verzweigte (C₁-C₆)-Dialkylaminogruppen oder 2,2-Dimethyl-1,3-dioxolan-4-yl-gruppen substituierte) Alkylgruppe bedeutet,
- und zwei Äquivalenten Triethylamin umsetzt,
so daß man nach der Abspaltung der Schutzgruppe in basischem Medium die Verbindung der Formel (I/a) erhält, einem Sonderfall der Verbindungen der Formel (I): 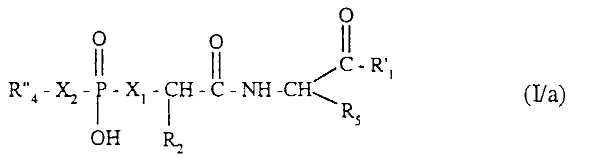 in der R'₁, R₂, R₅ und X₂ die oben angegebenen Bedeutungen besitzen und R"₄ eine (durch eine oder mehrere Hydroxy-, Benzyloxy-, Benzyloxycarbonylamino-, Acetoxy- oder geradkettige oder verzweigte (C₁-C₆)-Dialkylaminogruppen oder 2,2-Dimethyl-1,3-dioxolan-4-yl-gruppen substituierte) Alkylgruppe darstellt, bei welcher Verbindung der Formel (I/a) man die Gruppe R"₄, wenn diese;
- eine durch eine oder mehrere Benzyloxygruppen substituierte Alkylgruppe darstellt, man durch katalytische Hydrierung in eine durch eine oder mehrere Hydroxygruppen substituierte Alkylgruppe umwandeln kann,
- eine durch eine oder mehrere Acetoxygruppen substituierte Alkylgruppe darstellt, durch Umsetzen mit Lithiumhydroxid oder Natriumhydroxid in eine durch eine oder mehrere Hydroxygruppen substituierte Alkylgruppe umwandeln kann,
bei welcher Verbindung der Formel (I/a):
- man die Gruppe R'₁, wenn diese eine Alkoxygruppe darstellt, in die entsprechende Hydroxygruppe umwandeln kann,
- man mit Hilfe klassischer Reinigungsmethoden reinigen kann,
- man mit Hilfe klassischer Trennmethoden in die Isomeren auftrennen kann,
- und man indas entsprechende Säuren- oder Basensalz umwandeln kann.

6. Verfahren zur Herstellung von Phosphoramidon der Formel: 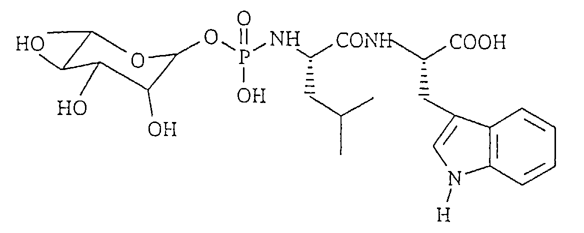 dadurch gekennzeichnet, daß es mit dem in Anspruch 5 beschriebenen identisch ist, wobei jedoch die Verbindung der Formel (VIII) durch die Verbindung der folgenden Formel ersetzt wird:

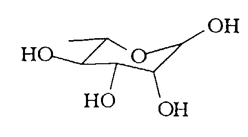

7. Pharmazeutische Zubereitung enthaltend als Wirkstoff eine Verbindung nach einem der Ansprüche 1 bis 4 allein oder in Kombination mit einem oder mehreren inerten, nichttoxischen, pharmazeutisch annehmbaren Trägermaterialien.

8. Pharmazeutische Zubereitung nach Anspruch 7 enthaltenen einen Wirkstoff nach einem der Ansprüche 1 bis 4 geeignet als Inhibitor von Endothelin-Convertase.
